# EUROPEAN PATENT APPLICATION

(11) **EP 4 104 879 A1**
(43) Date of publication of application: **21.12.2022**
(21) Application number: 22178945.6
(22) Date of filing: 14.06.2022
(51) Int. Cl.: A61M 5/158, A61M 25/00, A61M 5/32

(54) **IRREMOVABLE BUCKLING-RESISTANT FLEXIBLE CANNULA AND PROCESS**

(30) Priority: 18.06.2021 US 202117352154
(71) Applicant: Medtronic MiniMed, Inc., Northridge, CA 91325-1219 (US)
(72) Inventor: Tong, Davy, Thousand Oaks (US); Dang, Kiem H., Thousand Oaks (US); Fusselman, Hsi C., Simi Valley (US); Chattaraj, Sarnath, Simi Valley (US); Miller, Thomas P., Porter Ranch (US)
(74) Representative: Zimmermann & Partner Patentanwälte mbB

(57) **Abstract**

A cannula includes a tubular body having an axial dimension through which a flow channel extends. The tubular body has a first end for insertion into a subject, and a second end configured to be held within a base of a medical device. The tubular body has a first length portion extending along the axial dimension from the first end toward the second end. A second length portion extends along the axial dimension from the first length portion toward the second end, and has an outer dimension that flares outward to be larger than the outer dimension of the first length portion. The second length portion also has a tubing wall thickness that is greater than the tubing wall thickness of the first length portion.

## Description

### BACKGROUND

Various types of modern medical devices include or employ one or more cannula tubing for conveying fluid media. A cannula may be employed for conveying fluid media to or from a patient, a sensor, a pump, an insertion set or other medical device, a reservoir or fluid container, an implanted or partially implanted device, or the like. A cannula may be included in a sensor, a pump, in insertion set or other medical device. In some contexts, a cannula may be configured to be inserted into or partially into a patient, for example, through the patient's skin.

Certain diseases or conditions may be treated, according to modern medical techniques, by delivering a medication or other substance to the body of a user, through a cannula tubing, either in a continuous manner or at particular times or time intervals within an overall time period. For example, diabetes is commonly treated by delivering defined amounts of insulin to the user at appropriate times. Some common modes of providing insulin therapy to a user include delivery of insulin through manually operated syringes and insulin pens. Other modern systems employ programmable fluid infusion devices (e.g., insulin pumps or other fluid delivery devices) to deliver controlled amounts of insulin to a user. In certain instances, these infusion devices employ an infusion set (or an insertion set) having one or more cannula to be coupled to the body of a user for the delivery of the insulin.

Typically, the infusion set includes a cannula having a cannula portion that can be inserted under the skin of the user to deliver controlled amounts of infusion media to the user. Various examples of infusions sets that include a flexible tubing cannula are described in U.S. Patent Application Publication No. 2018/0318550 (Application No. 15/973,471 and U.S. Patent Application Publication No. 2020/0384187 (Application No. 16/436,486), which is incorporated herein by reference, in its entirety. Example cannulas as described herein may be employed with or included in those or other suitable infusion set devices. Example cannulas as described herein may be employed with other medical devices and systems for conveying fluid media to or from a patient, a sensor, a pump, an infusion set or other device or system.

For example, a cannula (or an infusion set with a cannula) as described herein may be configured or employed in an infusion media delivery system that includes a pump or other delivery device and an infusion media reservoir. In those systems, the cannula provides at least a portion of the fluid flow path to deliver the infusion media. In other examples, a cannula or an insertion set with a cannula may be configured in or employed in a sensor set, to couple a sensor to a body of the user. For example, a sensor set may be configured to monitor glucose levels, or to measure glucose levels in blood or interstitial fluid. In particular examples, an infusion set (or insertion set) includes at least one (or multiple) first cannula for delivery of infusion media or at least one second cannula for a sensor (or one or more of both first and second cannulas).

Further disclosed herein is a cannula that includes a tubular body having an axial dimension through which a flow channel extends, wherein the tubular body has a first end for insertion into a subject, and a second end configured to be held within a base of a medical device, wherein the tubular body has a first length portion extending along the axial dimension from the first end toward the second end, wherein a second length portion extends along the axial dimension from the first length portion toward the second end, and has an outer dimension that flares outward to be larger than the outer dimension of the first length portion, and wherein the second length portion also has a tubing wall thickness that is greater than the tubing wall thickness of the first length portion.

### BRIEF DESCRIPTION OF THE DRAWINGS

The above and other aspects and features of the present invention will become more apparent to those skilled in the art from the following detailed description of the example embodiments with reference to the accompanying drawings, in which:
Fig. 1 is a perspective view of an example of an infusion set having an example of a cannula.
Fig. 2 is a cross-section view of the base and upper housing portion of the infusion set example of Fig. 1.
Figure 3 is an exploded, perspective view of the cannula assembly and port member of the infusion set of Fig. 1.
Figure 4 is an enlarged cross-section view of the cannula and the needle guide of in a cannula receptacle of the infusion set of Fig. 1.
Figure 5 is a further enlarged cross-section view of the portion labeled (5) of Fig. 4.
Figure 6 is a partial, cut-away, perspective view of a cannula according to a further example.

### DETAILED DESCRIPTION

Hereinafter, example embodiments will be described in more detail with reference to the accompanying drawings. The present invention, however, may be embodied in various different forms, and should not be construed as being limited to only the illustrated embodiments herein. Rather, these embodiments are provided as examples so that this disclosure will be thorough and complete, and will fully convey the aspects and features of the present invention to those skilled in the art. Accordingly, processes, elements, and techniques that are not necessary to those having ordinary skill in the art for a complete understanding of the aspects and features of the present invention may not be described. Unless otherwise noted, like reference numerals denote like elements throughout the attached drawings and the written description, and thus, descriptions thereof may not be repeated. Further, features or aspects within each example embodiment should typically be considered as available for other similar features or aspects in other example embodiments.

Certain terminology may be used in the following description for the purpose of reference only, and thus are not intended to be limiting. For example, terms such as "top", "bottom", "upper", "lower", "above", and "below" could be used to refer to directions in the drawings to which reference is made. Terms such as "front", "back", "rear", "side", "outboard", and "inboard" could be used to describe the orientation and/or location of portions of the component within a consistent but arbitrary frame of reference which is made clear by reference to the text and the associated drawings describing the component under discussion. Such terminology may include the words specifically mentioned above, derivatives thereof, and words of similar import. Similarly, the terms "first", "second", and other such numerical terms referring to structures do not imply a sequence or order unless clearly indicated by the context.

It will be understood that when an element or feature is referred to as being "on," "connected to," or "coupled to" another element or layer, it can be directly on, connected to, or coupled to the other element or feature, or one or more intervening elements or features may be present. In addition, it will also be understood that when an element or features is referred to as being "between" two elements or features, it can be the only element or feature between the two elements or features, or one or more intervening elements or features may also be present.

The terminology used herein is for the purpose of describing particular embodiments and is not intended to be limiting of the present invention. As used herein, the singular forms "a" and "an" are intended to include the plural forms as well, unless the context clearly indicates otherwise. It will be further understood that the terms "comprises," "comprising," "includes," and "including," "has, " "have, " and "having," when used in this specification, specify the presence of the stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. Expressions such as "at least one of," when preceding a list of elements, modify the entire list of elements and do not modify the individual elements of the list.

As used herein, the term "substantially," "about," and similar terms are used as terms of approximation and not as terms of degree, and are intended to account for the inherent variations in measured or calculated values that would be recognized by those of ordinary skill in the art. Further, the use of "may" when describing embodiments of the present invention refers to "one or more embodiments of the present invention." As used herein, the terms "use," "using," and "used" may be considered synonymous with the terms "utilize," "utilizing," and "utilized," respectively.

Unless otherwise defined, all terms (including technical and scientific terms) used herein have the same meaning as commonly understood by one of ordinary skill in the art to which the present invention belongs. It will be further understood that terms, such as those defined in commonly used dictionaries, should be interpreted as having a meaning that is consistent with their meaning in the context of the relevant art and/or the present specification, and should not be interpreted in an idealized or overly formal sense, unless expressly so defined herein.

Example embodiments relate to cannula for conveying fluid media. Further example embodiments relate to infusion sets, insertion sets, media delivery systems, sensors or other medical devices and systems that include one or more cannula.

In certain examples, the cannula includes a hollow tube or tubing and is configured to be inserted into (or partially into) a patient, such as, through the patient's skin. In other examples, the cannula tubing is configured to be included in (or is included in) an implantable device configured to be implanted within the body, limb or head of a patient. The cannula may be included in an insertion set, an infusion set, a sensor device, an infusion pump or other fluid delivery system, or other medical device or system, or the like. In certain examples, the cannula may be connected to or included in an infusion set or an insertion set that has a base or hub configured to adhere to the skin of a patient. In other examples, the cannula may be connected to or included in another external medical device or in an implantable (internal) medical device.

The cannula according to certain examples may be configured to receive an insertion needle, for assisting with insertion of the cannula into or through a patient's skin, a septum or another structure. In some examples, the cannula tubing has a hollow channel in which the insertion needle is received and can slide. In other examples, the cannula tubing is configured to fit and slide inside the channel of a hollow needle. The needle can provide sufficient rigidity and a pointed or sharp end, for assisting with inserting the cannula through the patient's skin, the septum or the other structure. In particular examples, the needle is withdrawn, after insertion of the cannula, leaving the cannula extending through the patient's skin, the septum or the other structure.

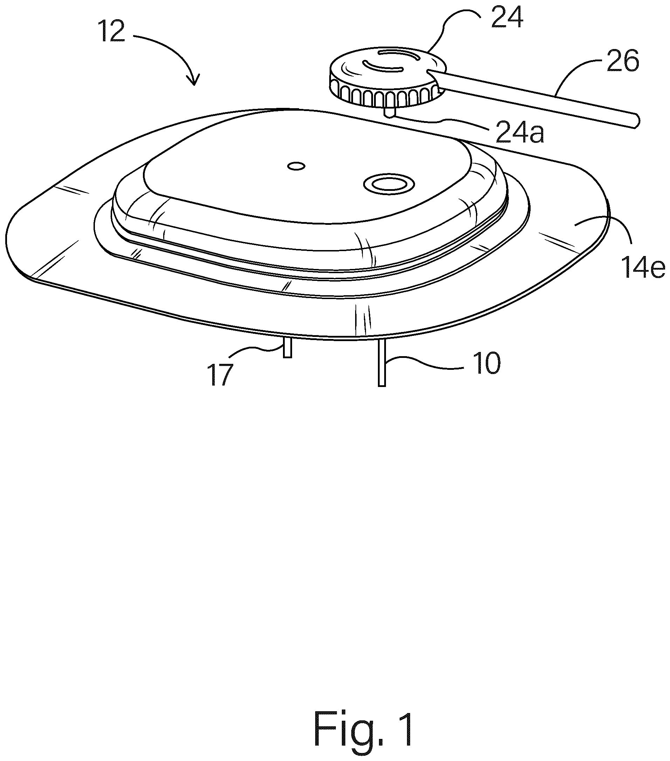

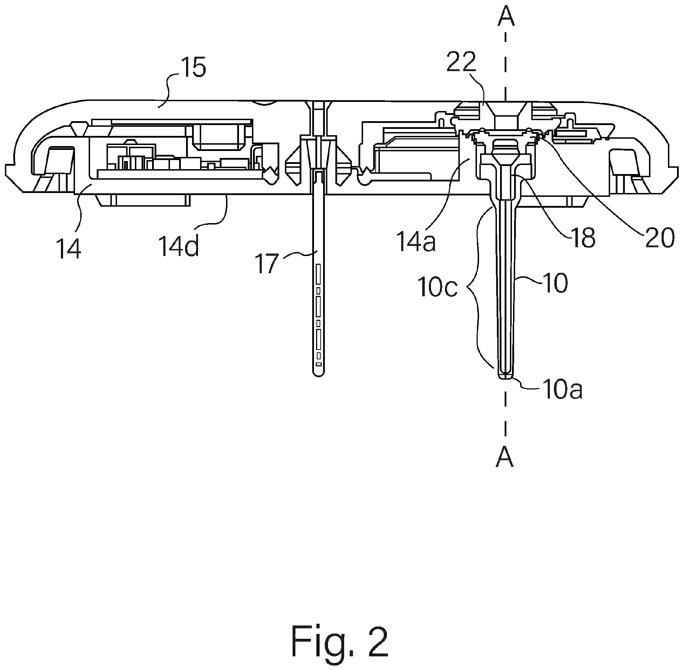

In the example in Figs. 1 and 2, a cannula 10 is included in infusion set 12 that is configured to be secured to a surface of a patient's skin (not shown). A perspective view of the infusion set 12 is shown in Fig. 1. A cross-section view of the infusion set 12 and cannula 10 is shown in Fig. 2.

The infusion set 12 includes the cannula 10 and a base 14 on which the cannula is held. In some examples, as shown in Figs. 1 and 2, the infusion set 12 may also include an upper housing portion 15 that is securable to the base by one or more suitable connection mechanisms such as, but not limited to snap fit, interference fit, adhesive, welding, clamps or other fasteners. In some examples, the upper housing portion 15 secures to the base 14, to form a housing for other components, for example, after the base 14 is secured to the patient's skin and after the cannula 10 is inserted into a patient. The base 14 and the upper housing portion 15 may be made of any material having sufficient rigidity to hold its shape and function as described herein, including, but not limited to plastic, metal, ceramic, composite material, combinations thereof, or the like.

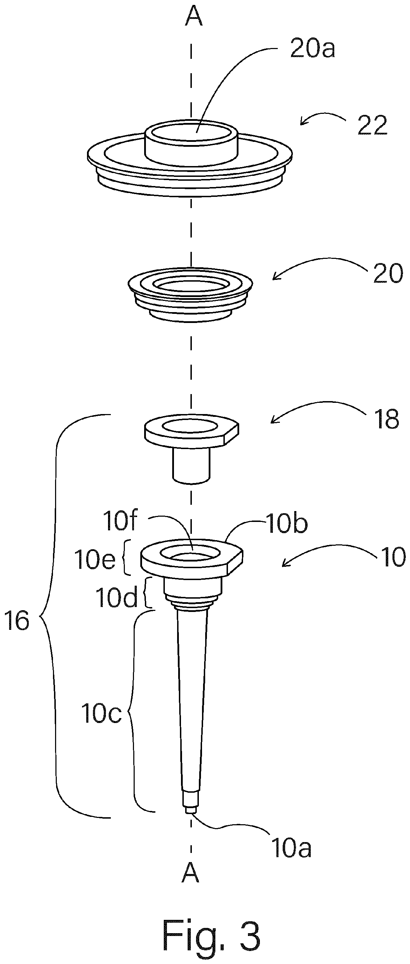

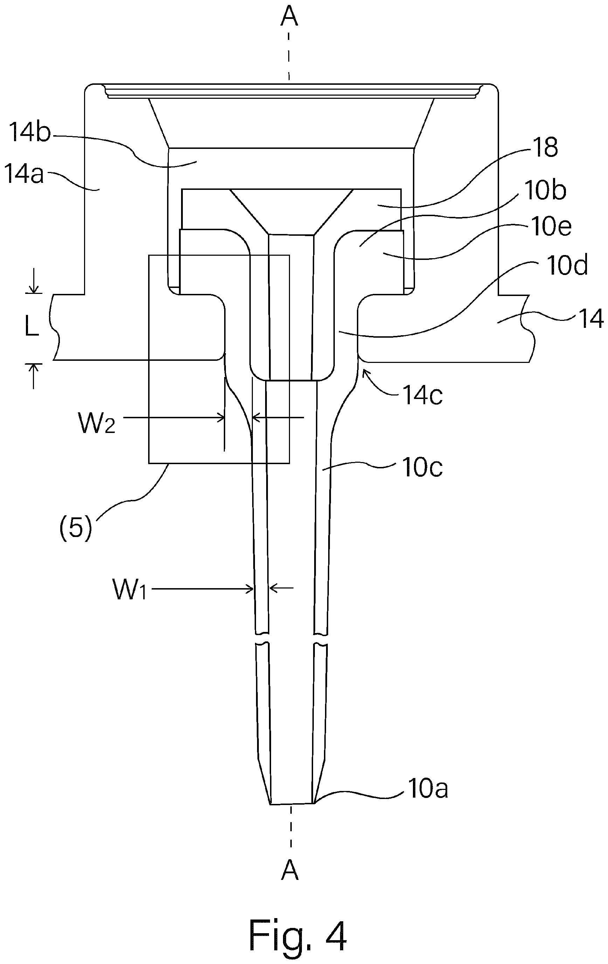

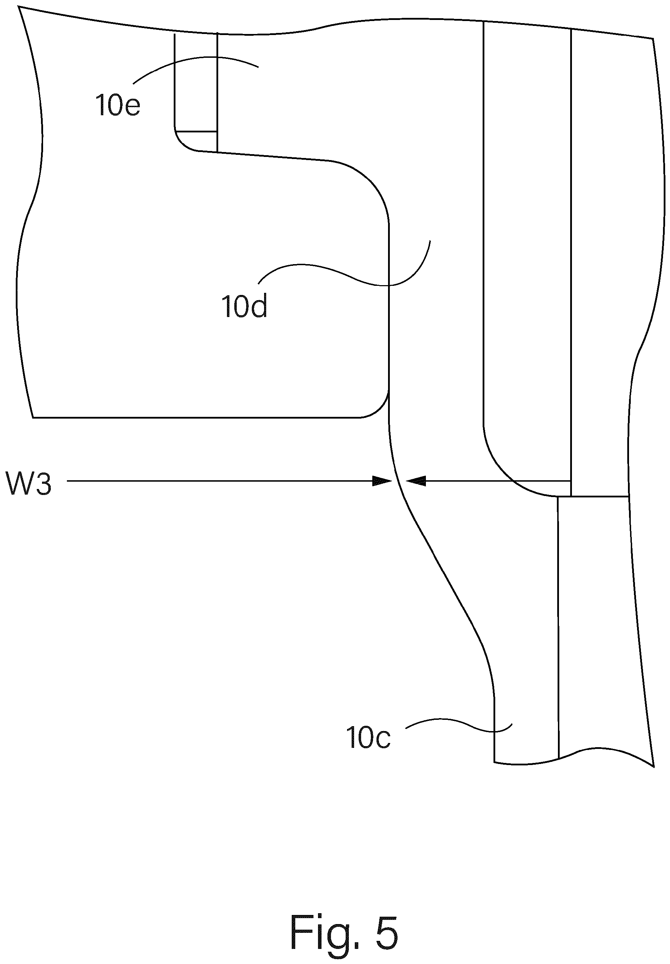

The cannula 10 may be part of a cannula assembly 16 that includes the cannula 10 and a needle guide 18. In some examples, the cannula assembly 16 may also include or operate with a septum 20 and a port member, as shown in an exploded view in Fig. 3. A cross-section view of the cannula assembly 16, as assembled with the base 14 of the infusion set 12, is shown in Fig. 2. An enlarged, cross-section view of the cannula 10 and the needle guide 20 in a receptacle 14a of the base 14 is shown in Fig. 4. Fig. 5 shows an enlarged view of a portion labeled (5) in Fig. 4.

In some examples, the infusion set 12 may include a port member 22 (Figs. 2 and 3) arranged over the septum 20 of the cannula assembly 16. The port member 22 has a central opening that provides an access port 22a for receiving a hollow needle or other insertable device to be inserted through the septum 20 to be in fluid flow communication with the cannula 10. The hollow tube 24a is in fluid flow communication with a fluid flow tubing 26 and passes through the septum 20 to connect the fluid flow tubing 26 in fluid flow communication with the cannula 10, when the connector hub 24 is placed on and connected to the rest of the infusion set 12, in the alignment shown in Fig. 1. The fluid flow tubing 26 may be a flexible tubing connected to a fluid delivery system, such as, but not limited to an infusion media pump and reservoir, for coupling the fluid delivery system in fluid flow communication with the cannula 10 of the infusion set 12. In other examples, the fluid delivery system may be included in the base 14 (or in the housing formed by the base 14 and the upper housing portion 15), or attaches onto the base 14 (for example, with the upper housing portion 15).

In the example in Fig. 2, the base 14 includes a receptacle 14a having a chamber 14b that receives and holds a portion of the cannula 10. Enlarged views of the receptacle 14a are shown in Figs. 4 and 5. The base 14 also has an opening 14c at the bottom of the receptacle 14a, through which a length portion of the cannula 10 extends, when the cannula is received in the chamber 14b. The opening 14c may have a diameter (or peripheral dimension) that is smaller than the inner diameter (or inner peripheral dimension) ID of the chamber 14b.

When received in the receptacle 14a of the base 14, the cannula 10 extends through the opening 14c and out from the base 14 (from the downward-facing surface of the base in Figs. 1 and 3). When the base 14 secured on or adjacent a patient's skin at a desired insertion site, the cannula 10 extends through the patient's skin to a desired insertion depth. In some examples, the cannula 10 is inserted into the receptacle 14a and extended through the opening 14c in the base (and into a patient's skin), by an action of an insertion tool, for example, at the time of (or after) the base 14 is secured to a patient's skin, and before the upper housing portion 15 is secured to the base 14. In other examples, the cannula 10 extends through the opening 14c in the base 14, before the base is secured to the patient's skin.

In certain examples, the surface 14d of the base 14 from which the cannula 10 extends (i.e., the downward-facing surface in Figs. 1 and 2) is configured to be secured to the skin of a patient. For example, an adhesive material (such as an adhesive pad 14e in Fig. 1) may be provided on that surface of the base 14, to secure the base 14 to the patient's skin at the desired insertion site (or on apparel, a band, a strap or another object at or adjacent the desired insertion site on the patient). In particular examples, the surface is provided with an adhesive that is sufficiently strong to hold the base or hub 14 on a patient's skin during operation of the infusion set 12, yet allow the patient (or medical staff) to selectively peel off and remove the base 14 from the patient's skin, for example, to inspect, service, or replace the infusion set 12, or to remove the infusion set 12 at the end of a treatment. Alternatively or in addition, the base 14 may operate with or include other mechanisms to secure the base 14 to the patient, including but not limited to sutures, bands, straps or the like.

In the illustrated example, the infusion set 12 has one cannula 10 coupled in fluid communication with a fluid source for infusion of fluid to a patient. In other examples, the infusion set may have more than one cannula for infusion of fluid, each connected to the same fluid source or respectively different fluid sources. The fluid source may include the tube 26 connected through the hub 24 and the port member 22, or may include a reservoir (not shown) located on the base 14.

In certain examples, the infusion set 12 may include one or more insertable sensor members configured to be inserted through the patient's skin, where each sensor member is connected to the same or different sensor electronics (not shown) located in the base 14. For example, in the example in Fig. 1, the infusion set includes a sensor cannula or sensor member 17. In other examples, the sensor cannula or sensor member 17 and sensor electronics may be omitted.

The cannula tubing having an axial length dimension (along axis A), a generally circular cross-section shape taken perpendicular to the axial length dimension, and a central passage through which fluid may flow. In other examples, the tubing may have a cross-section shape that is not a circle, such as, but not limited to an oval, another curved shape, a polygon or a shape having a combination of curved and straight edges. The cross-section shape of the central passage may have a circular or another shape, and may correspond to (be the same shape as) the outer cross-section shape of the tubing. In other examples, the cross-section shape of the central passage may be a different shape relative to the outer cross-section shape of the tubing.

The cannula tubing may be made of a material that is compatible with fluids intended to be conveyed through the tubing, and with other materials to which the tubing may come into contact or be connected, in the intended environment of use. In certain examples, the cannula tubing is made of a material that is biologically compatible, for use in contexts in which the tubing is in contact or connected with a biological entity (such as a human patient or another biological entity), or is implanted fully or partially in the patient (or other biological entity). In certain examples, the cannula tubing is treated in one or more processes for enhancing biologically compatible such as, but not limited to cleaning, sterilizing, coating with Heparin, or the like.

In certain examples the cannula tubing is made of a material that is suitable for medical uses, including but not limited to materials compatible with, and suitable for, implanting or partially implanting into a patient or other biological entity. Alternatively or in addition, the tubing material is selected to be compatible with and suitable for conveying one or more desired or predefined fluids (such as, but not limited to insulin, cancer or AIDs treatment media, pain treatment media, or other medications, drugs or therapy fluids). Such materials may include, but are not limited to an ethylene tetrafluoroethylene (ETFE), a polytetrafluoroethylene (PTFE), a fluorinated ethylene propylene (FEP), a perfluoroalkoxy (PFA), a polychloroprene (Neoprene), a polypropene (PP), a polypropylene, a polyethylene (PE), a fluorinated ethylene propylene (FEP) or other fluoropolymer, an ethylene vinyl acetate (EVA), a polyether block amide (PEBA) of thermoplastic elastomer (TPE) such as PEBAX^{™}, a thermoplastic polyurethane (TPU) such as PELLETHAN^{™}, a silicon material, other fluoropolymer, synthetic rubber, thermoplastic polymer, or the like. However, for other contexts and applications of use, the tubing may be made of other materials suitable and compatible with those contexts and applications. In particular examples in which the cannula tubing is made by injection molding, micromolding or other molding techniques, a tubing material compatible with such techniques.

The cannula tubing may be made by any suitable manufacturing process including, but not limited to extrusion, molding, machining, shrinking over a mandrel, combinations thereof, or the like. However, in particular examples, the tubing is made with or by a micromolding process as described herein. While various manufacturing processes may be employed, micromolding can be an efficient way to make a cannula shape and wall dimensions as described herein. In particular, micromolding can be used to provide a flared end with an increased wall thickness (or without reducing wall thickness by an amount that would compromise strength) of the cannula tubing.

The cannula tubing has a distal end 10a and a proximal end 10b that are open to the passage of the tubing. The axial length of the cannula tubing extends from the open distal end 10a to the open proximal end 10b. The distal end 10a and at least a portion of the length of the cannula 10 is configured to be inserted into a subject (patient's skin, or in other examples, a septum or other pierced structure). The proximal end 10b is configured to be connected to the base 14 (and is shown in Fig. 2 as received in and connected to the receptacle 14a of the base 14).

The tubing of the cannula 10 has an OD that changes along its length. As shown in Figs. 2 and 3, the tubing of the cannula 10 has a first length portion 10c, a second length portion 10d and a third length portion 10e. In particular examples, the first, second and third length portions 10c, 10d and 10e are formed together as a single, unitary structure, for example, by micromolding or other suitable process as described herein. In other examples, one or more of the length portions 10c, 10d, and 10e may be formed separate from the other length portions, and attached together with the other length portions by any suitable adhesive, weld, boding material, over-molded material or the like. The first length portion 10c extends from the distal end 10a to the second length portion 10d of the cannula tubing. The second length portion 10d extends from the first length portion 10c to the third length portion 10e of the cannula tubing. The third length portion 10e extends from the second length portion 10d to the proximal end 10b of the cannula tubing.

In the example in Fig. 2, some or all of the first length portion 10c extends out from a surface (the downward-facing surface in Figs. 1 and 2) of the base 14, to extend into a patient's skin, when the base 14 is secured to the patient and the cannula 10 is received in the receptacle 14a of the base 14. It can be desirable for the size of the outer dimension (or outer diameter) OD of the first length portion 10c of the cannula tubing to be relatively small (or minimized) for patient comfort, while still providing sufficient fluid flow capacity through the tubing. In other examples, other suitable dimensions may be employed, based on the context of use.

In particular examples, the OD of the cannula 10 is constant along the first length portion 10c. In other examples, the OD of the cannula 10 changes along the axial length of the first length portion 10c. Thus, in some examples, the OD of the cannula 10 may taper to a smaller OD at the distal end 10a, to a larger relative OD toward the second length portion 10d, as shown in Figs. 2 and 4. In some examples, a distal end section of the first length portion 10c adjacent the distal end 10a of the cannula 10 may be sharpened or narrowed, to reduce insertion force needed to pass the distal end through a skin surface, septum or other structure.

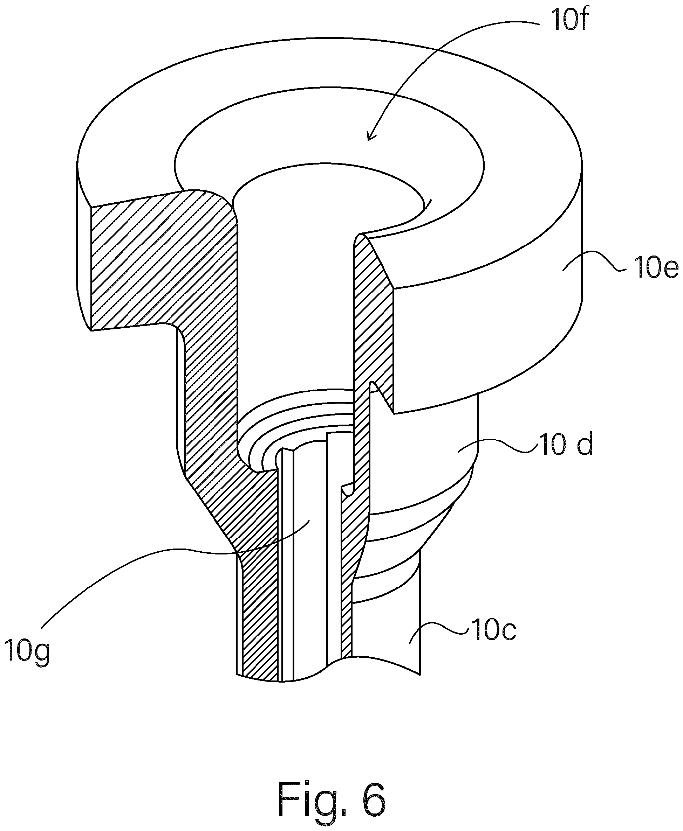

In particular examples, the ID of the cannula 10 is constant along the axial length of the first length portion 10c, from the distal end 10a to the second length portion 10d, as shown in Fig. 4. A constant ID along the first length portion 10c can provide an unrestricted flow channel and can simplify manufacture. In other examples described herein, the ID of the cannula 10 may include one or more features along its axial length, to inhibit kinking or buckling of the cannula tubing. In those or other examples, the ID or the OD (or both) of the cannula 10 may include one or more ribs, projections or other features along its axial length, to enhance rigidity or flexibility of the cannula tubing at one or more locations along the axial length. An example of ribs 10g for enhancing rigidity are discussed below with reference to Fig. 6.

In particular examples, the tubing wall may have a wall thickness or width W₁ that is relatively constant along the axial length dimension of the first length portion 10c. In some examples, the wall thickness or width W₁ of the first length portion 10c is constant from the distal end 10a to the second length portion 10d (or to a location at which the cannula 10 tapers to transition to the second length portion 10d). In other examples, the wall thickness or width W1 may vary along the first length portion 10c, for example, from a smaller width to a greater width in the axial direction from the distal end 10a toward the second length portion 10d.

The second length portion 10d of the cannula 10 has an OD and ID that flares or expands outward to a larger OD and ID relative to the OD and ID of the first length portion 10c. The flared ID of the second length portion 10d of the cannula provides a recess 10f for receiving the needle guide 18.

The second length portion 10d has an OD size and shape that is configured to be received at least partially in the opening 14c to the chamber 14b of the base 14. In particular examples, cannula material has resiliency and the OD of the second length portion 10d is larger (or slightly larger) than the ID of the opening 14c, such that the second length portion 10d is press fitted or interference fitted into the opening 14c of the base. In particular examples, the interference fit of the second length portion 10d in the opening 14c may be sufficient to provide a reliable liquid-tight seal. However, in certain examples, the interference fit is not tight enough to cause a compression of the cannula wall in the second length portion 10d to an extent that causes cold flow of the cannula wall material in the second length portion 10d or to the extent that causes great enough cold flow to be undesirable (e.g., to reduce pull strength below that of the first length portion 10c). Thus, in particular examples, the OD of the section L and the OD of the opening 14c are selected or controlled to provide a desired sealing effect, without undesirable cold flow of the cannula material. In some examples, the interference fit and sealing function of the second length portion 10d in the opening 14c is created or enhanced when the needle guide 18 is received in the recess 10f, as shown in Figs. 2 and 4.

In particular examples, the interference fit of the second length portion 10d includes engaging an outer surface of the second length portion 10d with an inner wall surface of the opening 14c. As shown in Figs. 2, 4 and 5, the engaged sealing surfaces are transverse to (such as, but not limited to perpendicular to) the axis A of the cannula. In particular examples, a length L of the transverse outer surface of the second length portion 10d engages the transverse inner wall surface of the opening 14c. The length L may be defined by the axial A length (or depth) of the opening 14c.

The OD of a length section L of the second length portion 10d is larger than the OD of the opening 14c by a selected amount W₃, to provide a level of compression along the section L of the second length portion 10d, for the interference fit. The amount W₃ of difference in the ODs may be dependent, at least in part, on the size of the cannula 10, and the cannula material. The length L of the engaged surfaces can affect the sealing ability, such that a longer length L may provide a greater sealing ability than a shorter length L. Accordingly, in certain examples, the depth of the opening 14c of the receptacle 14, and the axial length of the outer surface of the second length portion 10d of the cannula (and, thus, the length L of the engaged surfaces) is selected to provide a desired or an enhanced sealing effect.

Accordingly, for a given cannula material and size, the amount W3 and length L may be selected or configured appropriately, such that the sealing surface of the cannula 10 create a sufficient liquid seal, but need not be forced in an axial direction or compressed to an undesired cold-flow state. Instead, the length L of the sealing surface of the second length portion 10d presses radially outward, against the inner surface of the opening 14c, by the resilience of the cannula material or by the needle guide received in the recess 10f of the cannula 10 (or both).

The flared or expanded OD and the ID of the second length portion 10d of the cannula can be formed by any suitable manufacturing method including, but not limited to micromolding, other types of molding, machining, pressing, extruding, or combinations thereof. However, by micromolding, the shape and wall thickness or width dimensions can be controlled such that the thickness or width W₂ of the cannula wall in the second length portion 10d of the cannula is at least as great as (or greater than) the thickness or width W₁ of the cannula wall in the first length portion 10c.

By providing a wall thickness or width W₂ in the second length portion 10d that is greater than the thickness or width W₁ in the first length portion 10c, the pull strength of the second length portion 10d of the cannula in the base 14 may be increased. The wall thickness or width W₂ in the second length portion 10d is selected to provide a pull strength that is greater than the pull strength of the first length portion 10c. In particular examples, the wall thickness W₂ is selected such that the expanded or flared second length portion 10d (the portion that forms the seal) is sufficiently strong to not limit or reduce the overall pull strength of the cannula 10.

The third length portion 10e of the cannula 10 has an OD that is larger than the OD of the second length portion 10d. In the example in Figs. 2-4, the OD of the cannula abruptly changes (in a step-like manner) to a larger OD at the third length portion 10e. The third length portion 10e forms a lip or flange that extends radially outward relative to the outer dimension of the second length portion 10d. The lip or flange of the third length portion 10e surrounds the recess 10f, at the proximal end 10b of the cannula 10.

The third length portion 10e of the cannula 10 is located in the receptacle 14a of the base 14, when the cannula 10 is mounted on the base 14. The OD of the lip or flange of third length portion 10e is larger than the OD of the opening 14c in the base 14, such that the third length portion 10e of the cannula is inhibited from passing through the opening 14c, especially when the needle guide 18 is received in the recess 10f, as shown in Figs. 2 and 4.

The lip or flange of the third length portion 10e may have a circular cross-section shape (taken perpendicular to the axis A), defining a curved, circular outer circumference. In certain examples, one or more sections of the circular outer circumference (represented by the section 10g) may be un-curved or non-circular, to match a correspondingly shaped surface in the receptacle 14a of the base 14. More specifically, the receptacle 14a may have an inner surface with a curved, circular inner circumference (corresponding to and engaging the outer circumference of the lip or flange of the third length portion 10e), and may also include one or more un-curved or non-circular sections that match and mate with the one or more un-curved or non-circular sections of the lip or flange. Accordingly, when the third length portion 10e of the cannula 10 is received in the receptacle 14a of the base 14, the one or more un-curved or non-circular sections of the lip or flange of the cannula 10 engage the one or more un-curved or non-circular surfaces of the receptacle 14a, and inhibit rotation of the cannula 10 about the axis A relative to the base 14.

The recess 10f in the cannula 10 forms a cup-shaped receptacle for the needle guide 18. The bottom of the recess 10f (in the orientation of Figs. 2 and 4) is open to (in fluid flow communication with) the flow channel in the first length portion 10c of the cannula. The recess 10f has an inner surface that is shaped to match the outer surface of the needle guide 18, such that the needle guide 18 may fit snuggly and tightly in the recess 10f. When the needle guide 18 is fitted within the recess 10f, the central channel of the needle guide aligns in fluid flow communication with the opening at the bottom of the recess 10f and with the flow channel in the first length portion 10c of the cannula, as shown in Figs. 2 and 4.

Also as shown in Figs. 2 and 4, the second length portion 10d and the third length portion 10e of the cannula are received in the chamber 14b of the receptacle 14a in the base or hub 14, while the first length portion 10c of the cannula extends through and out of the opening 14c in the base or hub 14. In particular examples, the opening 14c in the base or hub 14 has an outer dimension (or diameter) OD that is the same or larger than the OD of the first length section 10c, but smaller than the maximum OD of the second length section 10d. Accordingly, when the cannula 10 is arranged in the base or hub 14, some or all of the first length portion 10c of the cannula extends out from the surface 14d of the base or hub 14, while some or all of the second length portion 10d is located within the chamber 14b, and while the third length portion 10e is within the chamber 14b. In that arrangement, because the maximum OD of the second length section 10d is larger than the OD of the opening 14c, the cannula 10 is retained in the base or hub 14 and inhibited from being pulled out or separated from the base or hub 14. Furthermore, in that arrangement, the needle guide 18 is received in the recess 10f and further helps to retain the cannula 10 in the base or hub 14.

The needle guide 18 includes a rigid body having a shaft section 18a extending from a lip or flange section 18b. The body of the needle guide 18 may be made of any material having sufficient rigidity to hold its shape and function as described herein, including, but not limited to plastic, metal, ceramic, composite material, combinations thereof, or the like.

The shaft and flange sections of the needle guide 18 form an annular body that has a central channel. The shaft section 18a has a generally cylindrical shape, while the lip or flange section 18b flares radially outward to a larger diameter relative to the shaft section 18a. In certain examples, the lip or flange section 18b has a generally circular outer circumference and one or more sections (represented by the section 18c) that are un-curved or non-circular, to match a correspondingly shaped surface in the receptacle 14a of the base 14. Accordingly, when the needle guide 18 is received in the recess 10f of the cannula 10, the one or more un-curved or non-circular sections of the lip or flange of the needle guide 18 engage one or more corresponding un-curved or non-circular surfaces in the receptacle 14a, and inhibit rotation of the needle guide 18 about the axis A relative to the base 14 and relative to the cannula 10.

The flange end of the needle guide 18 has an enlarged recess opening 18c (having a diameter that is larger than the diameter of the central channel of the needle guide, and tapers inward to the diameter of the central channel. The enlarged, tapered recess opening 18c of the needle guide 18 provides a guide for a needle or other insertable member, for receiving the needle or other insertable member in fluid flow communication with the proximal end 10b of the cannula 10.

As discussed above, the first length portion 10c of the cannula is configured to be inserted into a patient's skin. In particular examples, the cannula tubing has sufficient flexibility to allow the cannula tubing to flex or move with the patient's skin, for improved comfort. In addition, the cannula tubing may be sufficiently rigid to withstand kinking or buckling during insertion or when bent or curved. In particular examples, the cannula tubing flexibility and rigidity is selected and controlled by selecting or controlling one or more of the tubing dimensions and configuration (including its outside diameter OD and its inside diameter ID), the material from which the tubing is made, and the process of manufacture. In certain examples, the tubing may include one or more additional structural features to either enhance flexibility or to enhance rigidity (and resistance to kinking or buckling).

In some examples, the cannula tubing may be relatively flexible along its length, but is also configured to have a relatively high degree of flexibility (or enhanced flexibility) in one or more selected sections of the length of the tubing, to allow bending at a controlled location or in a controlled manner (or both). Alternatively or in addition, the cannula tubing may be configured to have a relatively high degree of rigidity (or enhanced rigidity) in one or more selected sections of the length of the tubing, to inhibit bending or buckling at a controlled location or in a controlled manner (or both). In other examples, the flexible tubing may be relatively rigid along sections of its length but have one or more sections of enhanced flexibility, to allow bending only or primarily within the one or more sections of enhanced flexibility. Examples of configurations that may be employed to provide one or more sections of enhanced flexibility along a length of a flexible tubing cannula include, but are not limited to those described in U.S. Patent Application Publication No. 2020/0384187 A1 (Application No. 16/436,496), which is incorporated herein by reference, in its entirety.

An example of a configuration providing one or more sections of enhanced rigidity (and resistance to buckling) is described with reference to Fig. 6, which shows a partial section view of an example of the cannula 10. The inner wall of the first length portion 10c of the cannula 10 in Fig. 6 includes one or more ribs 10g (one in view in Fig. 6). Each rib 10g is formed by a thicker wall portion that protrudes radially inward from the rest of the inner surface of the first length portion 10c. Each rib 10g may extend axially on the inner wall surface, along at least a section of the axial length of the first length portion 10c.

Cannula flexing can occur during or after insertion of the cannula. In some contexts, the cannula can tend to flex at the transition between the first length portion 10c and the second length portion 10d. Accordingly, in certain examples, each rib 10g extends from the second length portion 10d, toward the distal end 10a of the cannula, to provide enhanced resistance to buckling at in that portion of the cannula. In some examples, each rib 10g extends all of the way to the distal end 10a. In other examples, each rib 10g extends a portion, but not the entire length of the first length portion 10c. In particular examples, the one or more ribs 10g are located only on a section of the first length portion 10c that is adjacent the second length portion 10d, and extends toward the distal end 10a for about 5% to 40% of the length of the first length portion 10c. In other examples, the ribs extend from the second length portion 10d toward the distal end 10a for about 10% to 20% of the length of the first length portion 10c.

The flare or taper of the second length portion 10d of the cannula 10 may be formed by any suitable method. However, in some conventional tube flaring methods in which an end portion of a tubing is pressed into a flared shape, the tubing wall may stretch or cold flow (and be compromised in strength). In particular examples, a micromolding system or other molding system or procedure (or both) can be configured to form a wall thicknesses or widths of any desired dimensions along different portions of the length of the cannula. Micromolding or other molding systems and methods can be readily configured and controlled to define sufficiently precise cannula shapes and wall thickness dimensions, in an efficient manufacturing environment.

Alternatively a flared tubing end can be formed by other suitable processes, such as compressing or stretching an end portion over a mandrel or cone-shaped form, extruding, or machining. However, compressing or stretching processes tend to reduce the width or thickness of the tubing wall thickness or width. Accordingly, additional controls and variations of such other processes would be needed to form cannula shapes and wall thicknesses as described herein, which could increase manufacturing costs.

While various exemplary embodiments have been presented in the foregoing detailed description, it should be appreciated that a vast number of variations exist. It should also be appreciated that the exemplary embodiment or embodiments described herein are not intended to limit the scope, applicability, or configuration of the claimed subject matter in any way. Rather, the foregoing detailed description will provide those skilled in the art with a convenient road map for implementing the described embodiment or embodiments. It should be understood that various changes can be made in the function and arrangement of elements without departing from the scope defined by the claims, which includes known equivalents and foreseeable equivalents at the time of filing this patent application.

Further disclosed herein is the subject-matter of the following clauses:
1. A cannula comprising a tubular body having an axial dimension and through which a flow channel extends, the tubular body comprising:
   a first end for insertion into a subject, the first end being open in flow communication with the flow channel;
   a second end configured to be held within a base of a medical device,
   a first length portion extending along the axial dimension from the first end toward the second end, the first length portion having an outer dimension and at least one tubing wall thickness;
   a second length portion extending along the axial dimension from the first length portion toward the second end, the second length portion having an outer dimension that flares outward to be larger than the outer dimension of the first length portion, the second length portion having a tubing wall thickness that is greater than the at least one tubing wall thickness of the first length portion.
2. The cannula as recited in clause 1, wherein the first length portion has an outer dimension that is smaller at the first end and increases in size toward the second length portion.
3. The cannula as recited in clause 1 or in any of the preceding clauses, wherein the first length portion has an inner dimension that is constant along an axial dimension of the first length portion.
4. The cannula as recited in clause 1 or in any of the preceding clauses, wherein the first length portion has at least one feature for increasing the rigidity of at least a second of the first length portion.
5. The cannula as recited in clause 4, wherein the at least one feature comprises at least one axially extending rib on an inner surface of the flow channel in the first length portion.
6. The cannula as recited in clause 5, wherein the at least one axially extending rib comprises a plurality of axially extending ribs.
7. The cannula as recited in clause 1, wherein a section of the flow channel in the first length portion has at least one feature for increasing the rigidity of a section of the first length portion located adjacent the second length portion.
8. The cannula as recited in clause 7, wherein the at least one feature comprises at least one axially extending rib on an inner surface of the flow channel in the first length portion.
9. The cannula as recited in clause 1 or in any of the preceding clauses, wherein the second length portion has an outer surface that is configured to engage a surface of a medical device to provide a liquid seal, the outer surface being parallel to the axial dimension.
10. The cannula as recited in clause 1 or in any of the preceding clauses, wherein the second length portion has an inner dimension that is greater than the inner dimension of the first length portion and is configured to receive a needle guide.
11. The cannula as recited in clause 1 or in any of the preceding clauses, wherein the second length portion has a flared section that flares radially outward relative to the first length portion, and a further section that extends from the flared section, substantially parallel to the axial dimension.
12. The cannula as recited in clause 1 or in any of the preceding clauses, further comprising a third length portion extending along the axial dimension from the second length portion to the second end, the third length portion having an outer dimension that is greater than the outer dimension of the second length portion, the third length portion configured to be received in the medical device.
13. The medical device comprising the cannula of clause 1 or in any of the preceding clauses.
14. A medical device comprising:
   a base having a receptacle and an opening; and
   a cannula comprising a tubular body extending through the opening in the base and through which a flow channel extends, the tubular body having an axial dimension, the tubular body comprising:
      a first end for insertion into a subject, the first end being open in flow communication with the flow channel;
      a second end configured to be held within the receptacle of the base of a medical device;
      a first length portion extending along the axial dimension from the first end toward the second end, the first length portion having an outer dimension and at least one tubing wall thickness;
      a second length portion extending along the axial dimension from the first length portion toward the second end, the second length portion having an outer dimension that flares outward to be larger than the outer dimension of the first length portion, the second length portion having a tubing wall thickness that is greater than the at least one tubing wall thickness of the first length portion.
15. The medical device as recited in clause 14, wherein the first length portion has an outer dimension that is smaller at the first end and increases in size toward the second length portion.
16. The medical device as recited in clause 14 or in any of clauses 14-15, wherein the first length portion has at least one feature for increasing the rigidity of at least a second of the first length portion.
17. The medical device as recited in clause 16, wherein the at least one feature comprises at least one axially extending rib on an inner surface of the flow channel in the first length portion.
18. The medical device as recited in clause 14 or in any of clauses 14-17, wherein the second length portion has an outer surface that is configured to engage a surface of the opening in the base to provide a liquid seal, the outer surface being parallel to the axial dimension.
19. The medical device as recited in clause 14 or in any of clauses 14-18, further comprising a needle guide, wherein the second length portion has an inner dimension that is greater than the inner dimension of the first length portion, the needle guide being received in the inner dimension of the second length portion.
20. A method of making a cannula having a tubular body with an axial dimension and through which a flow channel extends, the method comprising:
   providing a mold for forming a tubular body having:
   a first end for insertion into a subject, the first end being open in flow communication with a flow channel;
   a second end configured to be held within a base of a medical device,
   a first length portion extending along the axial dimension from the first end toward the second end, the first length portion having an outer dimension and at least one tubing wall thickness;
   a second length portion extending along the axial dimension from the first length portion toward the second end, the second length portion having an outer dimension that flares outward to be larger than the outer dimension of the first length portion, the second length portion having a tubing wall thickness that is greater than the at least one tubing wall thickness of the first length portion; and
   adding a moldable material to the mold to form the cannula in the mold.

## Claims

1. A cannula comprising a tubular body having an axial dimension and through which a flow channel extends, the tubular body comprising:
a first end for insertion into a subject, the first end being open in flow communication with the flow channel;
a second end configured to be held within a base of a medical device,
a first length portion extending along the axial dimension from the first end toward the second end, the first length portion having an outer dimension and at least one tubing wall thickness;
a second length portion extending along the axial dimension from the first length portion toward the second end, the second length portion having an outer dimension that flares outward to be larger than the outer dimension of the first length portion, the second length portion having a tubing wall thickness that is greater than the at least one tubing wall thickness of the first length portion.

2. The cannula as recited in claim 1, wherein the first length portion has an outer dimension that is smaller at the first end and increases in size toward the second length portion, and/or wherein the first length portion has an inner dimension that is constant along an axial dimension of the first length portion.

3. The cannula as recited in any of the preceding claims, wherein the first length portion has at least one feature for increasing the rigidity of at least a second of the first length portion.

4. The cannula as recited in claim 3, wherein the at least one feature comprises at least one axially extending rib on an inner surface of the flow channel in the first length portion, wherein typically the at least one axially extending rib comprises a plurality of axially extending ribs.

5. The cannula as recited in claim 1, wherein a section of the flow channel in the first length portion has at least one feature for increasing the rigidity of a section of the first length portion located adjacent the second length portion.

6. The cannula as recited in claim 5, wherein the at least one feature comprises at least one axially extending rib on an inner surface of the flow channel in the first length portion.

7. The cannula as recited in any of the preceding claims, wherein the second length portion has an outer surface that is configured to engage a surface of a medical device to provide a liquid seal, the outer surface being parallel to the axial dimension, and/or wherein the second length portion has an inner dimension that is greater than the inner dimension of the first length portion and is configured to receive a needle guide, and/or wherein the second length portion has a flared section that flares radially outward relative to the first length portion, and a further section that extends from the flared section, substantially parallel to the axial dimension.

8. The cannula as recited in any of the preceding claims, further comprising a third length portion extending along the axial dimension from the second length portion to the second end, the third length portion having an outer dimension that is greater than the outer dimension of the second length portion, the third length portion configured to be received in the medical device.

9. The medical device comprising the cannula of any of the preceding claims.

10. A medical device comprising:
a base having a receptacle and an opening; and
a cannula comprising a tubular body extending through the opening in the base and through which a flow channel extends, the tubular body having an axial dimension, the tubular body comprising:
a first end for insertion into a subject, the first end being open in flow communication with the flow channel;
a second end configured to be held within the receptacle of the base of a medical device;
a first length portion extending along the axial dimension from the first end toward the second end, the first length portion having an outer dimension and at least one tubing wall thickness;
a second length portion extending along the axial dimension from the first length portion toward the second end, the second length portion having an outer dimension that flares outward to be larger than the outer dimension of the first length portion, the second length portion having a tubing wall thickness that is greater than the at least one tubing wall thickness of the first length portion.

11. The medical device as recited in claim 10, wherein the first length portion has an outer dimension that is smaller at the first end and increases in size toward the second length portion.

12. The medical device as recited in any of claims 10-11, wherein the first length portion has at least one feature for increasing the rigidity of at least a second of the first length portion, wherein typically the at least one feature comprises at least one axially extending rib on an inner surface of the flow channel in the first length portion.

13. The medical device as recited in any of claims 10-12, wherein the second length portion has an outer surface that is configured to engage a surface of the opening in the base to provide a liquid seal, the outer surface being parallel to the axial dimension.

14. The medical device as recited in any of claims 10-13, further comprising a needle guide, wherein the second length portion has an inner dimension that is greater than the inner dimension of the first length portion, the needle guide being received in the inner dimension of the second length portion.

15. A method of making a cannula having a tubular body with an axial dimension and through which a flow channel extends, the method comprising:
providing a mold for forming a tubular body having:
a first end for insertion into a subject, the first end being open in flow communication with a flow channel;
a second end configured to be held within a base of a medical device,
a first length portion extending along the axial dimension from the first end toward the second end, the first length portion having an outer dimension and at least one tubing wall thickness;
a second length portion extending along the axial dimension from the first length portion toward the second end, the second length portion having an outer dimension that flares outward to be larger than the outer dimension of the first length portion, the second length portion having a tubing wall thickness that is greater than the at least one tubing wall thickness of the first length portion; and
adding a moldable material to the mold to form the cannula in the mold.
